(19) 

Europäisches
Patentamt
European
Patent Office
Office européen
des brevets

(11) **EP 4 659 581 A1**

(12) **EUROPEAN PATENT APPLICATION**
published in accordance with Art. 153(4) EPC

(43) Date of publication:
**10.12.2025 Bulletin 2025/50**

(21) Application number: **24750053.1**

(22) Date of filing: **23.01.2024**

(51) International Patent Classification (IPC):
*A01N 59/02* (2006.01)   *A01P 3/00* (2006.01)
*A61L 2/18* (2006.01)   *C01B 15/08* (2006.01)
*C11D 3/395* (2006.01)

(52) Cooperative Patent Classification (CPC):
A01N 59/02; A01P 3/00; A61L 2/18; C01B 15/08;
C11D 3/395

(86) International application number:
**PCT/JP2024/001894**

(87) International publication number:
**WO 2024/162102 (08.08.2024 Gazette 2024/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.01.2023 JP 2023012657**

(71) Applicants:
• **KOMOTO, Tadashi**
  **Kiryu-shi, Gunma 376-0011 (JP)**

• **Suya, Yuriko**
  **Sagamihara-shi, Kanagawa 252-0315 (JP)**
• **Takatsu, Shinichi**
  **Toyota-shi, Aichi (JP)**

(72) Inventors:
• **KOMOTO Tadashi**
  **Kiryu-shi, Gunma 376-0011 (JP)**
• **SUYA Yuriko**
  **Sagamihara-shi, Kanagawa 252-0315 (JP)**

(74) Representative: **Dehns**
  **10 Old Bailey**
  **London EC4M 7NG (GB)**

(54) **DECOMPOSITION AGENT SOLUTION FOR MICROORGANISMS AND PRODUCTION METHOD THEREFOR, DISINFECTION/STERILIZATION TREATMENT METHOD, AND DISINFECTION/STERILIZATION TREATMENT SYSTEM**

(57)     The present invention provides a decomposing agent aqueous solution for disinfecting and sterilizing microorganisms, which contains a peroxodisulfate compound as an oxidizing agent and has a pH in the range of 1 to 2 at a temperature of 20°C to 70°C. As a result, it becomes possible to provide a decomposing agent aqueous solution for infectious and non-infectious microorganisms, a disinfection and sterilization treatment method using the same, and a disinfection and sterilization treatment system, which enable not only disinfection but also sterilization of bacterial spores having the highest resistance to sterilization, safely, efficiently, and at low cost, by using a highly versatile oxidizing agent.

FIG.1

EP 4 659 581 A1

**Description**

Technical Field

The present invention relates to an aqueous decomposing agent solution for infectious and non-infectious microorganisms, a method for producing the same, a disinfection and sterilization treatment method, and a disinfection and sterilization treatment system.

Background Art

With regard to disinfection, the 17th Revision of the Japanese Pharmacopoeia, Reference Information, pages 2414-2416, stipulates that, as a disinfection method for bacteria and fungi, a log reduction value (LRV) of 3 log or more (i.e., >99.9%) is considered to have disinfection efficacy.

In addition, with regard to sterilization, the "Guidelines for Disinfection and Sterilization" (HERUSU PUBLISHING CO., INC., 1999) indicates a sterility assurance level (SAL) of $10^{-6}$.

Furthermore, in the document (Reference 9) titled "Method for Confirming Effectiveness in the Treatment of Infectious Waste" contained in the "Manual for Infectious Waste Treatment Based on the Waste Management Act" (March 2018, Ministry of the Environment, Environmental Regeneration and Material Cycles Bureau), the following are listed as biological indicators having the highest resistance to sterilization: (1) Bacillus stearothermophilus (ATCC 7953) and (2) Bacillus subtilis var. niger (ATCC 9372). In addition, the "Guidelines for Disinfection and Sterilization" ((HERUSU PUBLISHING CO., INC., 2020) recommends Bacillus atrophaeus ATCC 9372 as one of the biological indicators exhibiting the strongest resistance to sterilization methods.

Specifically, disinfection of linens contaminated with Category I infectious diseases includes, in addition to hot water disinfection (80°C for 10 minutes), immersion disinfection in a 0.05-0.1% aqueous sodium hypochlorite solution for 30 minutes. For certain Category II infectious diseases, immersion disinfection may be performed in a 2-3.5% glutaraldehyde solution or in a 0.55% aqueous phthalaldehyde solution for 10 minutes.

On the other hand, sterilization methods include the following physical sterilization methods: (1) thermal methods such as autoclave sterilization (high-pressure steam sterilization) and dry heat sterilization; (2) irradiation methods such as radiation sterilization using gamma rays or electron beams; and (3) filtration sterilization. In addition, chemical sterilization methods include: (1) ethylene oxide gas sterilization; (2) hydrogen peroxide low-temperature gas plasma sterilization; (3) low-temperature steam formaldehyde gas sterilization; (4) hydrogen peroxide gas low-temperature sterilization; and (5) chemical sterilants such as peracetic acid, glutaraldehyde, and phthalaldehyde.

Although these sterilization methods are widely used, there are issues that need improvement. For example, in autoclave sterilization, it is essential to ensure that no air remains inside the object to be sterilized, that the sterilization chamber is not overfilled, and that the sterilization temperature, humidity, and pressure are properly measured and that the instruments are regularly inspected.

In addition, although ethylene oxide gas sterilization can be performed at low temperatures, attention must be paid to its long sterilization time, various health hazards to operators, degradation of the quality of the object to be sterilized, and its flammability.

Hydrogen peroxide used under a high vacuum state, which acts as a highly reactive radical that is 100% ionized and kills microorganisms, is used in plasma sterilization; however, it presents problems such as the inability to sterilize cellulose, powders, and liquids, as well as low permeability into long and narrow luminal structures.

However, in medical institutions, the above-mentioned issues have remained unresolved for many years and persist to the present day. Therefore, there is a demand for the development of methods or devices that allow disinfection and sterilization to be performed not under special conditions but under atmospheric pressure and below 100°C, and moreover, in aqueous solutions with high safety, as well as chemical substances that do not cause health hazards when handled properly. Based on this concept, the present inventors earnestly conducted research and development on novel disinfectants and sterilants for microorganisms.

In Non-Patent Literature 1, titled "Sulfate Radical-Induced Disinfection of Pathogenic Escherichia coli O157:H7 via Iron-Activated Peroxodisulfate," a study was reported on the disinfection of pathogenic Escherichia coli in water treatment at 25°C using sulfate radical ions ($SO_4^{*-}$) generated by activation of peroxodisulfate ions ($S_2O_8^{2-}$) with ferrous ions ($Fe^{2+}$).

In Non-Patent Literature 1, ferrous ions similar to those used in the Fenton reaction are used to generate active sulfate radical ions from an aqueous potassium peroxodisulfate (KPS) solution at room temperature. However, in order to sterilize only pathogenic and non-pathogenic microorganisms that are attached or adsorbed onto the surfaces of medical devices, instruments, or various other solids, compounds that generate ferrous ions must coexist, resulting in an increased number of post-treatment steps, which is also undesirable from a cost standpoint.

However, no research or patent literature has been found on the disinfection of infectious and non-infectious microorganisms such as E. coli O157:H7, or on the sterilization of bacterial spores, by generating sulfate radical ions from peroxodisulfates under atmospheric pressure and below 100°C, particularly at room temperature, in a system that does not contain coexisting substances such as ferrous ions.

On the other hand, Non-Patent Literature 2 reports that in a 70°C aqueous solution of potassium peroxodisulfate (KPS), the natural polymer chitosan undergoes depolymerization, with its molecular backbone cleaved by sulfate radical ions generated from the thermal decomposition products of KPS, resulting in the formation of low-molecular-weight chitosan. It should be noted that, since KPS does not thermally decompose in aqueous solutions in the temperature range of 60°C to room temperature, Non-Patent Literature 2 contains no description whatsoever regarding the decomposition of chitosan in KPS aqueous solutions at room temperature.

Patent Literature 1 describes a 1% mixture of 50% potassium peroxodisulfate and 5% sulfamic acid in distilled water as one of various disinfectant mixtures related to nanobacteria associated with apatite mineral formation. However, Patent Literature 1 does not disclose any specific sterilization method. Furthermore, the technique disclosed in Patent Literature 1 was not capable of killing Bacillus stearothermophilus.

As described above, the present inventors diligently investigated domestic and foreign patent literature, and as far as they could determine, no reports have been found regarding methods for disinfection of infectious and non-infectious microorganisms and sterilization of bacterial spores using aqueous solutions of KPS or mixtures of KPS and alkaline compounds not only at temperatures above 60°C but also in the room temperature range below 60°C.

Prior Art Documents

Patent Literature

Patent Literature 1: JP-T-2002-519363

[Non-Patent Literature]

Non-Patent Literature 1: Environmental Science & Technology Letters, 4, 154-160 (2017)
Non-Patent Literature 2: Polymer Degradation and Stability, Vol. 75 (1), 73-83 (2002)

Summary of the Invention

Problems to be Solved by the Invention

The present invention has been made in view of the above circumstances, and aims to provide a decomposing agent aqueous solution for infectious and non-infectious microorganisms, which is capable of not only disinfecting but also sterilizing bacterial spores having the highest resistance to sterilization, more inexpensively, safely, and efficiently by using a highly versatile oxidizing agent, as well as to provide a disinfection and sterilization treatment method and a disinfection and sterilization treatment system using the same.

Means for Solving the Problems

The present invention has been made to solve the above-mentioned technical problems and is characterized by the following features.

Firstly, the decomposing agent aqueous solution of the present invention is a decomposing agent aqueous solution for disinfecting and sterilizing microorganisms, which comprises a peroxodisulfate compound as an oxidizing agent, and is characterized in that the pH at a temperature of 20°C to 70°C is in the range of 1 to 2.

Secondly, in the decomposing agent aqueous solution of the above-mentioned first aspect of the invention, it is preferable that the solution further comprises an alkaline compound.

Thirdly, in the decomposing agent aqueous solution of the above-mentioned first or second aspect of the invention, it is preferable that the concentration of the peroxodisulfate compound in the aqueous solution is 0.001 mol/L or more and not more than the saturation concentration.

Fourthly, in the decomposing agent aqueous solution of the above-mentioned first to third aspects of the invention, it is preferable that the pH three months after production of the decomposing agent aqueous solution is maintained at the same value as the pH at the time of production.

Fifthly, the method for producing a decomposing agent aqueous solution of the present invention is characterized in that an aqueous solution containing a peroxodisulfate compound, or an aqueous solution containing the peroxodisulfate compound and an alkaline compound, is heated under atmospheric pressure to a temperature in the range of 90°C to 100°C, and then cooled to a temperature in the range of 20°C to 70°C.

Sixthly, in the method for producing a decomposing agent aqueous solution of the above-mentioned fifth aspect of the invention, it is preferable that the pH of the aqueous solution after cooling is adjusted to be lower than the pH of the solution before heating, and to be in the range of 1 to 2.

Seventhly, the disinfection and sterilization treatment method of the present invention is a method for treating infectious and non-infectious microorganisms, comprising a step of performing disinfection and sterilization treatment on a treatment target including infectious and non-infectious microorganisms using any one of the decomposing agent aqueous solutions of the above-mentioned first to fourth aspects of the invention,

> wherein the infectious and non-infectious microorganisms are either in a state dissolved in water,
> or in a state of being attached or adsorbed to a solid surface that is not corroded by the decomposing agent aqueous solution.

Eighthly, the disinfection and sterilization treatment system of the present invention is a system for performing disinfection and sterilization treatment on a treatment target including infectious and non-infectious microorganisms using any one of the decomposing agent aqueous solutions of the above-mentioned first to fourth aspects of the invention, the system comprising:

> a decomposing agent aqueous solution supply unit configured to supply the decomposing agent aqueous solution prepared under predetermined conditions;
> a disinfection and sterilization treatment unit configured to immerse the treatment target in the decomposing agent aqueous solution and perform disinfection and sterilization treatment by a predetermined method;
> a solution recovery unit configured to recover the solution after the disinfection and sterilization treatment; and

a treatment target recovery unit configured to wash and dry the treatment target and recover the treatment target,

> wherein the decomposing agent aqueous solution prepared in the decomposing agent aqueous solution supply unit is recovered by the solution recovery unit after the treatment,
> and wherein the treatment target, after being treated in the disinfection and sterilization treatment unit, is recovered by the treatment target recovery unit.

Effects of the Invention

According to the decomposing agent aqueous solution for microorganisms, the method for producing the same, the disinfection and sterilization treatment method, and the disinfection and sterilization treatment system of the present invention, it is possible to disinfect infectious and non-infectious microorganisms efficiently and safely to a log reduction value (LRV) of greater than 3 (i.e., a reduction to one-thousandth or less of the initial microbial count), and to sterilize bacterial spores having the highest resistance to sterilization to an LRV of greater than 6 (i.e., a reduction to one-millionth or less of the initial microbial count), by using a more inexpensive and highly versatile oxidizing agent.

[Brief Description of the Drawings]

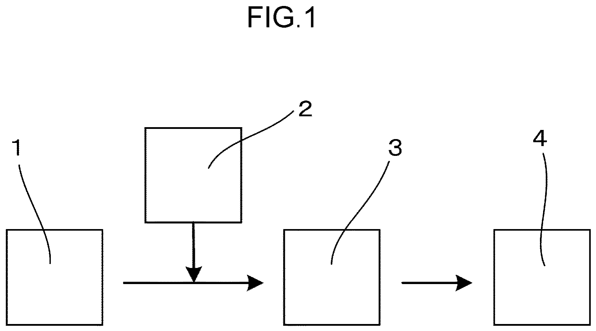

Fig. 1 is a schematic block diagram showing one embodiment of the disinfection and sterilization treatment system of the present invention.

Mode for Carrying Out the Invention

The present inventors conducted extensive research on decomposing agents capable of efficiently disinfecting and sterilizing infectious and non-infectious microorganisms. As a result, they found that an aqueous solution containing a peroxodisulfate compound as an oxidizing agent not only exhibits superior disinfection and sterilization performance compared to conventional chemical agents, but also can be used efficiently and safely under mild conditions of atmospheric pressure and 70°C or lower, and that neither the decomposition products of infectious and non-infectious microorganisms nor the decomposing agent remain on the surfaces of solids after disinfection, sterilization, and cleaning treatments. Based on these findings, the inventors completed the present invention.

The following provides a detailed description of embodiments of the decomposing agent aqueous solution for infectious and non-infectious microorganisms according to the present invention. The decomposing agent aqueous solution of the present invention is characterized by containing a peroxodisulfate compound as an oxidizing agent.

The peroxodisulfate compound used in the present invention is not particularly limited, and preferably used are water-soluble oxidizing agents such as potassium peroxodisulfate (KPS), sodium peroxodisulfate, and ammonium peroxodisulfate.

The decomposing agent aqueous solution of the present invention may further contain an alkaline compound. As the alkaline compound, those that are water-soluble and dissociate in the aqueous solution into a monovalent cation and a monovalent or divalent anion to exhibit alkalinity are preferably used. Examples of the alkaline compound include: alkali metal hydroxides such as sodium hydroxide, potassium hydroxide, and lithium hydroxide; alkali metal carbonates such as lithium carbonate, sodium carbonate, and potassium carbonate; alkali bicarbonates such as sodium bicarbonate and potassium bicarbonate; alkali metal oxalates such as sodium acetate and potassium oxalate; alkali metal succinates such as sodium succinate; and alkali metal salts of amino acids such as glycine, alanine, and glutamic acid. Among these, sodium bicarbonate and sodium hydroxide are particularly preferable.

The concentration of the peroxodisulfate compound (oxidizing agent) in the decomposing agent aqueous solution is not particularly limited, but is preferably 0.001 mol/L or more and not more than the saturation concentration. In addition, the pH of the aqueous solution before cooling is not particularly limited as long as it is in the range of 1 to 9.

The concentration of the alkaline compound in the decomposing agent aqueous solution is not particularly limited, but preferably, for example, the molar concentration ratio of the alkaline compound to the peroxodisulfate is in the range of 0 to 1.

Furthermore, the temperature of the decomposing agent aqueous solution is used in the range of 20°C to 70°C under atmospheric pressure. It should be noted that, in the present invention, "under atmospheric pressure" refers to a pressure equal to atmospheric pressure, which means approximately 1 atmosphere.

If the decomposing agent aqueous solution composed of a peroxodisulfate compound retains a predetermined concentration and temperature after disinfection and sterilization treatment, it is possible to subject the sterilized object to washing and drying with sterilized water (sterilized purified water) after removal. In addition, if the decomposing agent aqueous solution composed of a peroxodisulfate compound is not to be used further, it can be mixed with an alkaline compound such as an aqueous sodium bicarbonate solution and discarded as a safe, neutral saline solution such as sodium sulfate, which poses no environmental burden.

Representative examples of infectious and non-infectious microorganisms to be subjected to disinfection (LRV > 3) by the decomposing agent aqueous solution of the present invention include the following vegetative bacteria:

(1) Staphylococcus aureus (S. aureus);
(2) Methicillin-resistant Staphylococcus aureus (MRSA);
(3) Staphylococcus epidermidis (S. epidermidis);
(4) Methicillin-resistant Staphylococcus epidermidis (MRSE);

(5) Enterohemorrhagic Escherichia coli (EHEC):

1) E. coli O157:H7;
2) Pathogenic factors of EHEC include, in addition to verotoxins, adhesion factors such as intimin, which are involved in attachment to intestinal epithelial cells. EHEC is classified by serotypes based on O antigens present on the bacterial cell surface and H antigens present on the flagella. In Japan, the most common serotype is 0157, followed by O26 and O111.
3) Diarrheagenic Escherichia coli is classified into the following five types:

(1) Enteropathogenic Escherichia coli (EPEC);

(2) Enteroinvasive Escherichia coli (EIEC);

(3) Enterotoxigenic Escherichia coli (ETEC);

(4) Enteroaggregative Escherichia coli (EAEC);

(5) Enterohemorrhagic Escherichia coli (EHEC).

(6) Helicobacter pylori (H. pylori),

(7) Klebsiella pneumoniae (K. pneumoniae),

(8) Streptococcus pyogenes (S. pyogenes),

(9) Vancomycin-resistant enterococci (VRE),

(10) Campylobacter jejuni (C. jejuni),

(11) Shigella sonnei (S. sonnei),

(12) Vibrio cholerae (V. cholerae),

(13) Pseudomonas aeruginosa (P. aeruginosa),

(14) Multi-drug resistant Pseudomonas aeruginosa (MDRP),

(15) Multi-drug resistant Acinetobacter baumannii (MDRAB),

(16) Legionella pneumophila (L. pneumophila),

(17) Salmonella enterica (S. enterica),

(18) Typhoid (Salmonella enterica subsp. enterica serovar Typhi),

(19) Paratyphoid (Salmonella enterica subsp. enterica serovar Paratyphi A),

(20) Serratia marcescens (S. marcescens)

In addition, as test samples to be subjected to sterilization (LRV > 6), bacterial spores, which are biological indicators having the highest resistance to sterilization, include:

(1) Bacillus stearothermophilus (ATCC 7953),
(2) Bacillus subtilis var. niger (ATCC 9372), and
(3) Bacillus atrophaeus (ATCC 9372).

The method for producing the decomposing agent aqueous solution of the present invention is described below.

The decomposing agent aqueous solution of the present invention is produced by heating, under atmospheric pressure, an aqueous solution containing a peroxodisulfate compound as an oxidizing agent or an aqueous solution containing the peroxodisulfate compound and an alkaline compound to a predetermined temperature, maintaining the temperature for a certain period of time, then cooling the solution, and adjusting it to a predetermined pH.

Specifically, first, an aqueous solution of a peroxodisulfate compound as an oxidizing agent, or a mixed aqueous solution of a peroxodisulfate compound and an alkaline compound, is prepared. In this case, the concentration of the peroxodisulfate compound is preferably 0.001 mol/L or more and not more than the saturation concentration, and more preferably in the range of 0.01 to 0.2 mol/L.

In addition, the concentration of the alkaline compound in the decomposing agent aqueous solution is not particularly limited, but preferably, for example, the molar concentration ratio of the alkaline compound to the peroxodisulfate compound is in the range of 0 to 1.

In the method for producing the decomposing agent aqueous solution of the present invention, first, the above-described aqueous solution is heated under atmospheric pressure. The heating temperature is in the range of 90°C to 100°C. Next, the heated aqueous solution is maintained at the above temperature for 30 minutes or more.

Subsequently, the aqueous solution is cooled to a temperature in the range of 20°C to 70°C. By this cooling, the pH of the aqueous solution is adjusted to be in the range of 1 to 2 to obtain the decomposing agent aqueous solution. The cooling temperature is set to a level at which the pH can be adjusted to fall within the above range. It is preferable that the pH of the decomposing agent aqueous solution is adjusted to be lower than the pH of the solution before heating.

In the present invention, a more reliable disinfection and sterilization effect can be obtained by using the disinfection and sterilization treatment method employing the decomposing agent aqueous solution produced by the above-described method of the present invention. The disinfection and sterilization treatment method of the present invention will be described in detail below. The disinfection and sterilization method of the present invention includes a step of performing disinfection and sterilization treatment on a treatment target including infectious and non-infectious microorganisms using the above-described decomposing agent aqueous solution of the present invention.

In the step of performing disinfection and sterilization treatment, it is preferable that the infectious and non-infectious microorganisms are in a state dissolved or dispersed in water, or in a state of being attached or adsorbed to a solid surface that is not corroded by the decomposing agent aqueous solution. In addition, the container used for disinfection and sterilization treatment must be made of a solid material that is not corroded by the decomposing agent aqueous solution.

Examples of solid materials that are not corroded by the decomposing agent aqueous solution include stainless steels such as SUS304 and SUS316; heat-resistant glass; and homopolymers such as polyethylene, polypropylene, cycloolefin polymers, polyacetal, nylon, polyphenylene ether, polyethylene terephthalate, polybutylene terephthalate, poly-L-lactic acid, polycarbonate, polyvinylidene fluoride, polytetrafluoroethylene, polyphenylsulfone, polyphenylene sulfide, polyetheretherketone, aromatic polyamide, polyimide, methyl vinyl silicone rubber, and polyurethane; as well as copolymers and blends of these, natural fibers such as cellulose and pulp, nonwoven fabrics, and composite materials such as fiber-reinforced resins containing carbon fibers and aromatic polyamide fibers.

The treatment target to which microorganisms are attached or adsorbed is not particularly limited, and examples include medical devices, textile products, films, various molded articles, instruments, and various containers. These treatment targets are subjected to the preparation, reaction, washing, and drying steps of the decomposing agent aqueous solution using containers and instruments made of stainless steel, glass, resin, or composite materials thereof, which are not corroded by the decomposing agent aqueous solution, thereby completing the disinfection and sterilization operation.

Furthermore, in the disinfection and sterilization treatment method of the present invention, the decomposition treatment in the decomposing agent aqueous solution can be carried out in a stationary state, and may also be carried out, as appropriate, with stirring or ultrasonic irradiation.

The time for treating the treatment target in the decomposing agent aqueous solution (i.e., the time for decomposing infectious and non-infectious microorganisms) can be appropriately selected depending on the concentration of the oxidizing agent, the composition of the decomposing agent, and the temperature, and is not particularly limited.

Furthermore, in the present invention, a disinfection and sterilization treatment system using the above-described decomposing agent aqueous solution of the present invention can be constructed. One embodiment of the disinfection and sterilization treatment system of the present invention will be described in detail below with reference to the drawing.

In the disinfection and sterilization treatment system of the present invention, an aqueous peroxodisulfate solution or a mixed aqueous solution of a peroxodisulfate compound and an alkaline compound is used as the decomposing agent aqueous solution. Disinfection and sterilization treatment is performed on infectious and non-infectious microorganisms attached to the treatment target in a container that has resistance to decomposition by these aqueous solutions. After the treatment is completed, neutralization treatment may be carried out as necessary so that the waste solution can be rendered safe and environmentally friendly. Furthermore, in the disinfection and sterilization treatment system of the present invention, the treatment target can be washed and dried with sterilized water (sterilized purified water) within the above-mentioned pressure and temperature range.

As the disinfection and sterilization treatment system of the present embodiment, the configuration shown in Fig. 1 is illustrated. Specifically, the disinfection and sterilization treatment system includes: a decomposing agent aqueous solution supply unit 1 configured to supply a decomposing agent aqueous solution prepared under predetermined conditions; a disinfection and sterilization treatment unit 2 configured to immerse a treatment target to be disinfected and sterilized in the decomposing agent aqueous solution and perform disinfection and sterilization treatment by a predetermined method; a solution recovery unit 3 configured to recover the solution after disinfection and sterilization treatment; and a treatment target recovery unit 4 configured to wash, filter, and dry the treatment target and then recover it. The decomposing agent aqueous solution prepared in the decomposing agent aqueous solution supply unit 1 can be recovered by the solution recovery unit 3 after the treatment, and the treatment target, after being treated in the disinfection and sterilization treatment unit 2, can be recovered by the treatment target recovery unit 4.

In the disinfection and sterilization treatment system of the present embodiment, various containers exemplified in the above-described disinfection and sterilization treatment method can be used as instruments for supplying and storing the decomposing agent aqueous solution in the decomposing agent aqueous solution supply unit 1, and for accommodating the treatment target, adjusting the temperature of the aqueous solution, and immersing the treatment target in the decomposing agent aqueous solution in the disinfection and sterilization treatment unit 2, where disinfection and sterilization reactions are carried out. In the solution recovery unit 3, which recovers the solution after disinfection and sterilization treatment, and the treatment target recovery unit 4, which washes, filters, dries, and recovers the treatment target, commonly known instruments for solution recovery, washing, and drying can be used, and additionally, for example, stainless steel baskets may also be used together. The shape, size, and the like of the above containers and instruments are not particularly limited and may be appropriately selected according to the size and other characteristics of the treatment target. Furthermore, the decomposing agent aqueous solution supply unit 1 may be provided with mechanisms for preparing the raw peroxodisulfate aqueous solution or peroxodisulfate, as well as heating and cooling mechanisms for the aqueous solution.

By means of the disinfection and sterilization treatment system of the present invention, it is possible to efficiently carry out the preparation and storage of the decomposing agent aqueous solution, as well as disinfection and sterilization, and also to safely and efficiently recover the solution after treatment and the treatment target.

[Examples]

The present invention will be described in more detail below with reference to examples. However, the present invention is not limited to the following examples.

As indicated in Non-Patent Literature 1, it is known that in the presence of iron ions, as in the Fenton reaction, potassium peroxodisulfate (hereinafter also simply referred to as KPS) generates sulfate ion radicals in an aqueous solution at room temperature, and these radicals exhibit disinfection and sterilization activity against microorganisms. However, when metal ions and the like are present, a washing and removal step for such ions is required after disinfection and sterilization.

The present inventors have discovered a method for generating stable ion radicals from KPS in an aqueous solution over a wide temperature range, including room temperature, without containing active substances such as iron ions, in a colorless, odorless, and transparent aqueous system. Furthermore, the present inventors have also discovered a novel method for disinfecting and sterilizing infectious and non-infectious microorganisms through the action of these ion radicals. These effects were confirmed through the following examples.

(Example 1)

(Temperature dependence of the pH of KPS aqueous solution)

In Example 1, the temperature dependence of a KPS aqueous solution was examined. Specifically, under conditions in which molecular chains of natural polymers (vegetative bacteria and bacterial spores) are decomposed by the action of sulfate ion radicals generated from KPS in an aqueous solution at 70°C, the pH of the aqueous solution was measured under the conditions shown in Table 1 to evaluate the change in pH with temperature. The results are shown in Table 1. From these results, it was confirmed that the pH of the KPS aqueous solution at 70°C is 2.2.

It was also found that an aqueous solution of KPS at a concentration of 0.1 mol/L has a pH of 5.3 at room temperature (20°C), pH 5.1 at 25°C, pH 3.4 at 37.5°C, pH 2.7 at 55°C, and decreases to pH 1.1 at 90°C.

[Table 1]

| No. | KPS Concentration (mol/L) | Reaction Conditions and pH of Aqueous Solution |
|-----|---------------------------|------------------------------------------------|
| 1 | 0.1 | Room temperature (20°C), approx. pH 5.3 |
| 2 | 0.1 | Heated to 25°C from room temperature, approx. pH 5.1 |
| 3 | 0.1 | Heated to 37.5°C from room temperature, approx. pH 3.4 |
| 4 | 0.1 | Heated to 55°C from room temperature, approx. pH 2.7 |
| 5 | 0.1 | Heated to 70°C from room temperature, approx. pH 2.2 |
| 6 | 0.1 | Heated to 85°C from room temperature, approx. pH 1.6 |
| 7 | 0.1 | Heated to 90°C from room temperature, approx. pH 1.1 |

Furthermore, the pH of the KPS aqueous solution after heating at 90°C for 30 minutes and subsequent cooling to a predetermined temperature was measured. Specifically, under atmospheric pressure, the KPS aqueous solution was heated from room temperature to 90°C, maintained at that temperature for 30 minutes, and then cooled to temperatures of 25°C, 55°C, and 70°C, respectively. The pH of the KPS aqueous solution was measured at each temperature. The results are shown in Table 2.

That is, the present inventors succeeded in preparing an aqueous solution of sulfate ion radicals derived from KPS, which had not been obtainable by conventional techniques at temperatures below 70°C, even in the room temperature range.

[Table 2]

| No. | KPS Concentration (mol/L) | Reaction Conditions and pH of Aqueous Solution |
|---|---|---|
| 1 | 0.1 | Heated from room temperature to 90°C / 30 min → cooled to 25°C, approx. pH 1.9 |
| 2 | 0.1 | Heated from room temperature to 90°C / 30 min → cooled to 55°C, approx. pH 1.2 |
| 3 | 0.1 | Heated from room temperature to 90°C / 30 min → cooled to 70°C, approx. pH 1.6 |

(Example 2)

In Example 1 above, since each aqueous solution exhibited a low pH, a disinfection and sterilization test was next conducted using the KPS aqueous solution subjected to the heating and cooling treatment. In Example 1, when KPS aqueous solutions were prepared by heating at 90°C for 30 minutes and then cooling to a predetermined temperature, and subsequently stored for a long period of over three months at room temperature in sealed glass containers, it was found that the pH remained almost unchanged in all cases.

The disinfection and sterilization test methods for infectious and non-infectious microorganisms using the decomposing agent aqueous solutions of Example 1 and Example 2 of the present invention will be described below together with examples. However, the decomposing agent aqueous solutions and the disinfection and sterilization test methods of the present invention are not limited to the following examples in any way. The decomposing agent aqueous solution is effective either as KPS alone or as a mixed aqueous solution of KPS and an alkaline compound.

(Disinfection Test Method)

A disinfection test was conducted, as described below, against enterohemorrhagic Escherichia coli O157:H7, one of the vegetative bacteria, as a representative biological indicator for bacterial and fungal disinfection methods recognized in the 17th Revision of the Japanese Pharmacopoeia, Reference Information 2414-2416.

As culture media, (1) Tryptic Soy Agar (Difco, hereinafter referred to as TSA) and (2) SCDLP broth medium were used. As the reagent for preparing the media, sodium chloride (0.85% physiological saline solution) was used.

As the test microorganism, enterohemorrhagic Escherichia coli O157:H7 (Escherichia coli RIMD 0509939, a verotoxin-producing strain) was used. The test bacterial suspension was prepared by inoculating the frozen stock strain onto TSA and culturing at 36 ± 2°C for 24 hours. Then, a second inoculation onto the same medium was performed, followed by incubation at 36 ± 2°C for 18 hours. Subsequently, the grown colonies were scraped off and suspended in sterilized ion-exchanged water, and adjusted to approximately $10^8$ CFU/mL, which was used as the test bacterial suspension.

As the method for preparing the test solutions, KPS aqueous solutions (0.1 mol/L or 0.01 mol/L) were prepared in glass Erlenmeyer flasks and maintained at various predetermined temperatures; these were used as the test solutions. In addition, test solutions were also prepared by heating the aqueous solutions at 90°C for 30 minutes and then cooling them to various temperatures, after which the solutions at those temperatures were used as the test solutions.

In the bactericidal efficacy test, 0.1 mL of the test bacterial suspension was added to 10 mL of the test solution, mixed, and allowed to act at a predetermined temperature for a predetermined period of time. After the specified exposure time, 1 mL of the test solution was added to 9 mL of inactivating agent (SCDLP) to stop the bactericidal activity against the test microorganism, and this was used as the sample solution for bacterial count measurement. For the 0-minute (initial) sample and the control, sterilized physiological saline was used instead of the test solution.

For bacterial count measurement, the sample solution was used as the undiluted stock, and a 10-fold serial dilution series was prepared using physiological saline. For each 1 mL of undiluted and diluted sample solution, it was transferred to a Petri dish, mixed with approximately 20 mL of TSA, and solidified. The plates were then incubated at 36 ± 2°C for 43 hours. After incubation, the number of grown colonies was counted, and the number of test bacteria per 1 mL of test solution was determined (quantification limit: 10 CFU/mL).

The log reduction value (LRV) was calculated from the initial number of test bacteria and the number of bacteria remaining after exposure to the test solution, using the following Equation (1). The LRV was expressed as one decimal place (the second decimal place was rounded down).

LRV (log reduction value) = $\log_{10}$ (initial bacterial count in control / bacterial count after exposure to test solution)    (Equation 1)

Regarding the disinfection of pathogens, the 17th Revision of the Japanese Pharmacopoeia, Reference

9

Information 2414-2416 stipulates that a disinfection effect is considered present when the LRV (log reduction value) of the test microorganism is 3 log or more (>99.9%).

(Sterilization Test Method)

A sterilization test was conducted, as described below, against Geobacillus stearothermophilus *(ATCC* 7953) and Bacillus atrophaeus (ATCC 9372), which are the most resistant biological indicators among infectious and non-infectious microorganisms.

As culture media, (1) Tryptic Soy Agar (Difco, hereinafter referred to as TSA) and (2) SCDLP broth medium were used. As the reagent for preparing the media, sodium chloride (0.85% physiological saline solution) was used.

As the test microorganisms, Geobacillus stearothermophilus ATCC 7953 (spore suspension manufactured by CROSSTEX, Lot. AR579, approx. $10^9$ CFU/mL, sterilization indicator spore-forming bacterium) or Bacillus atrophaeus ATCC 9372 (spore suspension manufactured by NAMSA, Lot. N35105, approx. $10^9$ CFU/mL, or spore suspension manufactured by CROSSTEX, Lot. BT324, approx. $10^9$ CFU/mL, dry heat sterilization indicator bacterium) were used, and the undiluted spore suspensions were used in the tests.

As the method for preparing the test solution, a predetermined amount of sterilized distilled water was dispensed into a glass container, and the decomposing agent was dissolved therein.

In the bactericidal efficacy test, after preparing the test solution, the solution was maintained within $\pm 2°C$ of the test temperature, and 0.1 mL of the test bacterial suspension was added and mixed, and allowed to act for a predetermined period of time. After the specified period, 1 mL of the mixture was added to 9 mL of SCDLP, which had been confirmed to be an effective inactivating agent, to stop the bactericidal activity against the test microorganism. This was used as the sample solution for bacterial count measurement. For the control, physiological saline was used in place of the test solution and subjected to the same procedure.

Bacterial count measurement was performed as follows. The sample solution for bacterial count measurement was used as the undiluted stock, and a 10-fold serial dilution series was prepared using physiological saline. For each 1 mL of the undiluted and diluted sample solutions, the solution was transferred to a Petri dish, mixed with approximately 20 mL of TSA, and allowed to solidify. Bacillus atrophaeus was then incubated at $36 \pm 2°C$, and Geobacillus stearothermophilus at $52 \pm 2°C$, for 48 hours. In addition, all of the remaining volume of the undiluted KPS test solution was filtered through a membrane filter with a pore size of 0.45 $\mu$m (Millipore), and the filter was placed on a TSA plate and cultured in the same manner as above. After incubation, the number of grown colonies was counted, and the number of bacteria per 1 mL of the sample solution was determined (quantification limit: 1 CFU/mL).

The log reduction value (LRV) and reduction rate were calculated using the initial bacterial count and the bacterial count after exposure to the test solution, based on Equation (1) described above.

Here, an LRV of 6 or greater indicates that the number of bacteria in the sample solution has been reduced to one millionth or less by the decomposing agent aqueous solution, that is, the decomposing agent aqueous solution possesses excellent sterilization performance.

(Disinfection Test for Infectious and Non-Infectious Microorganisms)

The disinfection tests for infectious and non-infectious microorganisms in Examples 3 and 4 and Comparative Example 1 will be described in detail below. The disinfection test conditions and results for E. coli O157:H7 using KPS aqueous solutions in Examples 3 and 4 and Comparative Example 1 are shown in Table 3.

(Example 3)

In order to perform a disinfection test for enterohemorrhagic Escherichia coli O157:H7 under mild conditions for a short period of time, the methods described in Examples 1 and 2 were applied. First, a decomposing agent aqueous solution with a KPS concentration of 0.1 mol/L (pH 1) was prepared by heating at 90°C for more than 30 minutes, and then cooled to 55°C to obtain the decomposing agent aqueous solution (pH 1.2). Using this decomposing agent aqueous solution, a disinfection test was conducted, and a high LRV value of >5.6 was obtained. That is, a sterilizing effect far exceeding LRV >3.0 was achieved.

(Example 4)

Except for setting the KPS decomposing agent concentration at 0.01 mol/L, the disinfection test was conducted under milder conditions than in Example 3. In this case, the methods described in Examples 1 and 2 were applied. A KPS aqueous solution (room temperature, pH 5.4) was heated at 90°C for 30 minutes (pH 1.7), then cooled to 25°C to prepare a KPS decomposing agent aqueous solution (pH 1.9). A disinfection test for enterohemorrhagic Escherichia coli O157:H7 was conducted using this decomposing agent aqueous solution (25°C, pH 1.9) for 5 minutes, resulting in an LRV greater than 3.0. Thus, disinfection was possible at a low concentration of 0.01 mol/L KPS under mild conditions of 25°C for 5 minutes. Furthermore, in the post-treatment, the sulfate ion radicals activated from KPS were neutralized with a sodium bicarbonate aqueous solution to form sodium sulfate, enabling detoxification by a simple and safe method such as filtration and rinsing.

In general, disinfection targets are not limited to aqueous solutions of vegetative bacteria alone; rather, a wide variety of products are included, such as numerous fibrous materials and products thereof, mixtures containing powdered substances, and solid materials made from various components.

In the case of disinfection of vegetative bacteria dispersed in aqueous solutions containing such diverse materials, shapes, densities, and dispersion states as described above, it is assumed that the frequency of collisions between the bacteria and the sterilizing agent, i.e., the reactivity, will differ from that in aqueous solutions consisting solely of bacteria. Therefore, longer reaction times are preferable compared to disinfection of bacterial suspensions alone. Accordingly, in disinfection systems that include not only bacteria but also solid materials, a disinfection time several times longer than the aforementioned mild conditions of 25°C for 5 minutes is preferable.

Such an increase in reaction time also applies to the sterilization reaction described later.

(Comparative Example 1)

Except for omitting the preheating at 90°C for 30 minutes and the cooling step of the 0.01 mol/L KPS aqueous solution, the procedure was the same as in Example 4. KPS was directly dissolved in distilled water at 25°C to prepare a 0.01 mol/L KPS aqueous solution, which was then maintained at the same temperature for 30 minutes. Next, a disinfection test for enterohemorrhagic Escherichia coli O157:H7 was conducted in the KPS aqueous solution (pH 5.1) at 25°C for 5 minutes. As a result, LRV = 0 was observed, indicating that no disinfection effect occurred. Therefore, it was demonstrated that although a KPS aqueous solution prepared at 25°C cannot be directly applied to disinfection or sterilization, as in Example 4, when the KPS aqueous solution is first held at 90°C for 30 minutes and then cooled or allowed to cool naturally to 25°C to obtain a KPS decomposing agent aqueous solution (approximately pH 1.9), disinfection of enterohemorrhagic Escherichia coli O157:H7 can be performed within 5 minutes.

[Table 3]

|  | KPS Concentration (mol/L) | Reaction Conditions (Temperature, Time) | pH | LRV | Result |
|---|---|---|---|---|---|
| Example 3 | 0.1 | 90°C, heated for 30 min → cooled to 55°C, 5 min | 1.2 | >5.6 | Significant disinfection effect |
| Example 4 | 0.01 | 90°C, heated for 30 min → cooled to 25°C, 5 min | 1.9 | 3.8 | Significant disinfection effect |
| Comparative Example 1 | 0.01 | 25°C, 5 min | 5.1 | 0 | No disinfection effect |

(Disinfection Test of Decomposing Agent Aqueous Solution Containing Alkaline Compound)

Next, as Examples 5 and 6, a disinfection test was conducted under the following conditions for the decomposition of enterohemorrhagic Escherichia coli O157:H7 in a mixed aqueous solution (decomposing agent aqueous solution) of KPS and sodium bicarbonate as an alkaline compound. The disinfection test conditions and results for enterohemorrhagic Escherichia coli O157:H7 are shown in Table 4.

(Example 5)

Sodium bicarbonate was used as the alkaline compound, and the disinfection test was conducted in the same manner as in Example 3, except that the KPS concentration/sodium bicarbonate concentration was set to 0.1 mol/L / 0.05 mol/L. As a result, a high LRV value of >5.6, the same as in Example 3, was obtained, confirming that a high disinfection effect was also achieved even when the alkaline compound was included.

(Example 6)

Sodium bicarbonate was used as the alkaline compound, and the disinfection test was conducted in the same manner as in Example 4, except that the KPS concentration/sodium bicarbonate concentration was set to 0.01 mol/L / 0.005 mol/L. As a result, a high LRV value of >3.9, exceeding the threshold of LRV >3.0 for determining the effectiveness of the disinfectant, was obtained, confirming that a high disinfection effect was also achieved even when the alkaline compound was included.

[Table 4]

| | KPS Concentration / NaHCO$_3$ Concentration (mol/L) | Reaction Conditions (Temperature, Time) | pH | LRV | Result |
|---|---|---|---|---|---|
| Example 5 | 0.1 / 0.05 | 90°C, heated for 30 min → cooled to 55°C, 5 min | 1.2 | >5.6 | Disinfection |
| Example 6 | 0.01 / 0.005 | 90°C, heated for 30 min → cooled to 25°C, 5 min | 1.9 | >3.9 | Disinfection |

As described above, since the disinfection tests using KPS aqueous solution and a mixed aqueous solution of KPS and sodium bicarbonate against enterohemorrhagic Escherichia coli O157:H7 achieved an LRV > 3.0 (i.e., the number of viable bacteria was reduced to one one-thousandth or less), it can be said that the same approach is also applicable to the group of 20 types of vegetative bacteria represented by the aforementioned enterohemorrhagic Escherichia coli O157:H7.

(Sterilization Test of Bacterial Spores at 70°C or Higher)

A sterilization test accompanying the decomposition of bacterial spores in a mixed aqueous solution of KPS and sodium bicarbonate was conducted under conditions of atmospheric pressure and a temperature of 70°C to 100°C.

(Reference Example 1)

A decomposing agent aqueous solution containing 0.2 mol/L of potassium peroxodisulfate (KPS) was prepared in a heat-resistant glass container (beaker), and 0.1 mL of a Bacillus atrophaeus (ATCC9372) bacterial suspension was added to 9 mL of the decomposing agent aqueous solution heated to 85°C to prepare a test solution. The number of inoculated bacteria was $2.2 \times 10^7$ CFU per 1 mL of test solution. The solution was left to stand at the above temperature for 60 minutes to allow the decomposing agent to act. Then, 1 mL of the test solution was added to and mixed with 9 mL of SCDLP medium whose effectiveness as an inactivating agent had been confirmed, thereby stopping the activity of the test solution and measuring the number of viable bacteria. All remaining test solution was filtered using a 0.45 μm membrane filter, and after cultivation, the number of colonies formed was counted to determine the number of bacteria per 1 mL of the sample solution (quantification limit: 1 CFU/mL). As a result, an LRV > 6.4 was obtained, revealing that the decomposing agent aqueous solution exhibited high sterilization performance against Bacillus atrophaeus.

(Reference Examples 2 and 3)

Sterilization tests were conducted using a decomposing agent aqueous solution of KPS at 0.2 mol/L (Reference Example 2), and a mixed aqueous solution of KPS ($K_2S_2O_8$) at 0.2 mol/L and sodium bicarbonate (NaHCO$_3$) at 0.1 mol/L (molar ratio 2:1) (Reference Example 3), each with an initial bacterial count of $2.2 \times 10^7$ CFU per 1 mL of test solution. The sterilization tests were conducted at 95°C and 85°C, respectively. The results are shown in Table 5. As a result, an LRV > 6.3 was obtained in the sterilization tests, confirming that the decomposing agent aqueous solution has high sterilization performance.
In addition, for Geobacillus stearothermophilus ATCC7953, sterilization tests were also conducted in the same manner as in Reference Examples 1 to 3, and in all cases, an LRV > 6.2 was obtained, confirming that the decomposing agent aqueous solution has high sterilization performance.

[Table5]

| Reference Example | Bacterial Spore | $K_2S_2O_8$ (mol/L) | $NaHCO_3$ (mol/L) | Reaction Temperature (°C) | Reaction Time (min) | LRV |
|---|---|---|---|---|---|---|
| Reference Example 2 | Bacillus atrophaeus | 0.2 | 0 | 95 | 60 | >6.3 |
| Reference Example 3 | | 0.2 | 0.1 | 85 | 60 | >6.3 |

(Sterilization test at a fixed temperature of 70°C or lower after heating at 90°C or higher for 30 minutes or more.)

Next, a sterilization test of bacterial spores was conducted in a KPS decomposing agent aqueous solution that had been heated at 90°C or higher for 30 minutes or more and then cooled and maintained at 70°C or lower.

The bacterial count after the sterilization test was measured in the same manner as in Reference Example 1: the entire remaining volume of the test solution was filtered through a membrane filter with a pore size of 0.45 μm, and the number of colonies grown after incubation was counted to determine the number of bacteria per 1 mL of the sample solution (quantification limit: 1 CFU/mL). The LRV value was then obtained based on the result.

(Example 7)

A sterilization test was conducted using a KPS aqueous solution with a concentration of 0.1 mol/L that had been prepared at room temperature, held at 90°C for 30 minutes (pH 1.4), and then cooled and maintained at 70°C (pH 2.2). When Bacillus atrophaeus was subjected to this solution for 60 minutes, a high LRV value of greater than 6.2 was obtained.

(Example 8)

A sterilization test was conducted in the same manner as in Example 7, except that the KPS concentration was adjusted to 0.05 mol/L. After heating the solution at 90°C for 30 minutes and then cooling and maintaining it at 70°C for 60 minutes, a sterilization test was performed on Bacillus atrophaeus. Under these conditions, the pH of the aqueous solution was 1.8. As a result, an LRV of 5.5 was obtained. This value indicates that the number of viable bacteria was reduced to approximately 3 parts per million of the initial bacterial count.

The "Guideline on Sterilization Assurance in Clinical Practice 2015" (published by the Japanese Society for Medical Instrumentation, pp. 138-139) describes biological indicators (BIs) as a direct means to verify the microbiological sterilization efficacy of a sterilization process. The required performance for these BIs, as established in international standards, is $1.0 \times 10^5$ CFU or more for steam sterilization, $1.0 \times 10^6$ CFU or more for ethylene oxide gas sterilization, and $1.0 \times 10^5$ CFU or more for low-temperature steam formaldehyde gas sterilization.

As described in the Background Art section, the sterilization method of the present invention possesses characteristics superior to the three conventional sterilization methods mentioned above. Furthermore, it is significant that a high sterilization count of $10^{5.5}$ CFU or more was demonstrated. This value corresponds to a sterility assurance level of $10^{-6}$. Accordingly, even when cooled to 70°C, the solution retains high sterilization performance. Moreover, it is evident that by using a KPS concentration higher than 0.1 mol/L, an LRV of greater than 6 can be achieved within 60 minutes.

(Example 9)

A sterilization test was conducted under the same conditions as in Example 7, except that the bacterial species was Geobacillus stearothermophilus. As a result, LRV> 6.2 was obtained, demonstrating a high sterilization effect.

(Example 10)

A sterilization test was conducted under the same conditions as in Example 8, except that the bacterial species was Geobacillus stearothermophilus. As a result, LRV> 6.2 was obtained, demonstrating a high sterilization effect.

(Example 11)

A sterilization test was conducted under the same conditions as in Example 7, except that the bacterial species was Bacillus atrophaeus, and that a 0.1 mol/L KPS aqueous solution was held at 90°C for 30 minutes (pH 1.4), then cooled to 55°C and maintained at that temperature for 24 hours. As a result, even at 55°C, which is lower than 70°C, a high sterilization performance of LRV>6.4 was obtained, thereby demonstrating the novelty of the present invention.

(Example 12)

Furthermore, a sterilization test was conducted under the same conditions as in Example 11, except that the concentration of KPS in the aqueous solution was set to 0.05 mol/L. As a result, it was demonstrated that even at 55°C and with a lower concentration of KPS aqueous solution than in Example 11, a high sterilization performance of LRV>6.4 was achieved.

(Example 13)

A sterilization test was conducted under the same conditions as in Example 11, except that the bacterial species was Geobacillus stearothermophilus. As a result, an LRV >6.6 was obtained, demonstrating a high sterilization effect.

(Example 14)

A sterilization test was conducted under the same conditions as in Example 13, except that the concentration of KPS in the aqueous solution was set to 0.05 mol/L. As a result, a high sterilization performance of LRV>6.6 was obtained even with the KPS aqueous solution at a lower concentration than in Example 13, revealing that the present invention also has high sterilization performance against bacterial spores at 55°C, thereby demonstrating its novelty.

(Example 15)

A sterilization test was conducted under the same conditions as in Example 11, except that Bacillus atrophaeus was used as the bacterial species, the concentration of KPS was 0.2 mol/L, the sterilization test temperature after heating and cooling was set to a lower temperature of 37.5°C, and the test time was set to 48 hours. As a result, a high sterilization performance of LRV>6.4 was obtained.

(Example 16)

A sterilization test was conducted under the same conditions as in Example 15, except that the concentration of KPS in the aqueous solution was 0.1 mol/L. As a result, a high sterilization performance of LRV>6.4 was obtained.

(Example 17)

A sterilization test was conducted under the same conditions as in Example 15, except that the bacterial species was Geobacillus stearothermophilus and the test duration was 24 hours. As a result, a high sterilization performance of LRV>6.6 was obtained.

(Example 18)

A sterilization test was conducted under the same conditions as in Example 17, except that the KPS concentration in the aqueous solution was set to 0.1 mol/L. As a result, a high sterilization performance of LRV>6.6 was obtained even at 37.5°C.

(Example 19)

A sterilization test was conducted under the same conditions as in Example 15, except that the bacterial species was Bacillus atrophaeus, the sterilization test temperature after heating and cooling was set to 25°C, and the test duration was set to five days. As a result, a high sterilization performance of LRV > 6.4 was obtained.

(Example 20)

Except that the KPS concentration in the aqueous solution was set to 0.1 mol/L, a sterilization test was conducted under the same conditions as in Example 19. As a result, it was found that, although the reaction time was five days, the KPS aqueous solution at room temperature with a low concentration exhibited high sterilization performance against Bacillus atrophaeus, with an LRV > 6.4.

(Example 21)

A sterilization test was conducted under the same conditions as in Example 19, except that the bacterial species was Geobacillus stearothermophilus. Even when the type of bacterial spore was changed, it was demonstrated that the KPS aqueous solution of the present invention retained its activity as an ionic radical for a long period at room temperature (25°C) after heating at 90°C for 30 minutes and cooling, and exhibited high sterilization performance with an LRV > 6.6.

(Example 22)

A sterilization test was conducted under the same conditions as in Example 21, except that the KPS concentration in the aqueous solution was further reduced to 0.1 mol/L. This aqueous solution also achieved high sterilization performance with an LRV > 6.6. From the above results, it was revealed that the sterilizing agent aqueous solution of the present invention enables the disinfection of enterohemorrhagic Escherichia coli O157:H7 by a reaction of only 5 minutes at a low concentration (0.01 mol/L) as shown in Comparative Example 1, and further enables the sterilization of bacterial spores within up to 5 days in an aqueous solution with a KPS concentration of 0.1 mol/L. The conditions and results of Examples 7 to 22 are shown in Table 6.

[Table 6]

| Example | Bacterial Spore | $K_2S_2O_8$ (mol/L) | Reaction Temperature (°C) | Reaction Time | LRV |
|---|---|---|---|---|---|
| Example 7 | B. atro. | 0.1 | 70 | 60 min | 6.2 |
| Example 8 | B. atro. | 0.05 | 70 | 60 min | 5.5 |
| Example 9 | G. stearo. | 0.1 | 70 | 60 min | >6.2 |
| Example 10 | G. stearo. | 0.05 | 70 | 60 min | >6.2 |
| Example 11 | B. atro. | 0.1 | 55 | 24 h | >6.4 |
| Example 12 | B. atro. | 0.05 | 55 | 24 h | >6.4 |
| Example 13 | G. stearo. | 0.1 | 55 | 24 h | >6.6 |
| Example 14 | G. stearo. | 0.05 | 55 | 24 h | >6.6 |
| Example 15 | B. atro. | 0.2 | 37.5 | 48 h | >6.4 |
| Example 16 | B. atro. | 0.1 | 37.5 | 48 h | >6.4 |
| Example 17 | G. stearo. | 0.2 | 37.5 | 24 h | >6.6 |
| Example 18 | G. stearo. | 0.1 | 37.5 | 24 h | >6.6 |
| Example 19 | B. atro. | 0.2 | 25 | 5 days | >6.4 |
| Example 20 | B. atro. | 0.1 | 25 | 5 days | >6.4 |
| Example 21 | G. stearo. | 0.2 | 25 | 5 days | >6.6 |
| Example 22 | G. stearo. | 0.1 | 25 | 5 days | >6.6 |

Note: $K_2S_2O_8$ : KPS, B. atro. : Bacillus atrophaeus, G. stearo. : Geobacillus stearothermophilus
Reaction Temperature: After preparing the KPS aqueous solution at room temperature, the solution was heated at 90°C for 30 minutes, then cooled to the temperature indicated and used for the sterilization test.

(Change in LRV over Time)

Page 26 of the "Guidelines for Sterilization Assurance in Medical Settings 2021" (Japan Association for the Advancement of Medical Equipment) presents Appendix 1D titled "Establishment of Sterilization Conditions," which includes a "logarithmic death curve" (a semilogarithmic relationship between the logarithm of the number of viable microorganisms and sterilization time).
The reaction conditions shown in Table 6 above correspond to reaction times longer than the time required to achieve LRV = 6. After the minimum sterilization time corresponding to LRV = 6, the LRV value becomes constant.
Since the KPS aqueous solutions shown in Table 6, after heating and cooling, were maintained at constant temperatures and sustained a pH in the range of 1-2 over a long period, tests were conducted - mainly for Bacillus atrophaeus - around the time point where LRV = 6 is achieved.

(Example 23 and 24)

As a result, it was found that, in a KPS aqueous solution cooled and maintained at 70°C after heating from room temperature to 90°C for 30 minutes, the change in LRV value with respect to the reaction time of Bacillus atrophaeus (at concentrations of 0.05 mol/L and 0.1 mol/L) increased linearly until reaching LRV=6 (Table 7). It should be noted that, at concentrations higher than 0.1 mol/L of the sterilizing agent, the LRV value subsequently became slightly higher than LRV=6.

[Table 7]

| Change in LRV over Time at a Sterilization Temperature of 70°C after Heating and Cooling | | | LRV | | | |
|---|---|---|---|---|---|---|
| | Bacterial Spore | KPS Concentration (mol/L) | 0 min | 30 min | 60 min | 90 min |
| Example 23 | B. atro. | 0.05 | 0 | 4.1 | 5.5 | 5.5 |
| Example 24 | | 0.1 | 0 | 4.2 | 6.2 | 6.0 |

(Examples 25 and 26)

For Bacillus atrophaeus and Geobacillus stearothermophilus (both at a concentration of 0.05 mol/L), as in the previous section, each sterilization test was conducted using a KPS aqueous solution that had been heated and held, then cooled and maintained at 55°C. The results obtained for Examples 25 and 26 are shown in Table 8. For Bacillus atrophaeus, the LRV value increased linearly until it exceeded 6, and it was found that LRV = 6 was reached in 3.2 hours. After that, since all bacteria were killed, the LRV value remained constant. Therefore, it was clarified that the time required to achieve complete sterilization of Bacillus atrophaeus was 3.2 hours. Similarly, in the case of Geobacillus stearothermophilus, LRV = 6 was reached in 2.7 hours.

From these results, it was revealed that under the same conditions, Bacillus atrophaeus has greater resistance to sterilization than Geobacillus stearothermophilus. However, it was also clarified that both types of bacterial spores can be completely sterilized in approximately 4 hours under relatively mild conditions of 55°C in a KPS aqueous solution with a concentration of 0.05 mol/L.

[Table 8]

| Change in LRV over time at a sterilization temperature of 55°C after heating and cooling | | | LRV | | | |
|---|---|---|---|---|---|---|
| Example | Bacterial Spore | KPS Concentration (mol/L) | 0 h | 2 h | 3 h | 24 h |
| Example 25 | B. atro. | 0.05 | 0 | 4.1 | 5.8 | >6.4 |
| Example 26 | G. stearo. | 0.05 | 0 | 4.2 | >6.6 | >6.6 |

Furthermore, as in the previous section, a sterilization test of Bacillus atrophaeus was conducted in aqueous KPS solutions with concentrations of 0.05 mol/L and 0.2 mol/L, which had been heated and held, then cooled and maintained at a low temperature of 40°C. The change in LRV over time was examined, and the results for Examples 27 and 28 are shown in Table 9.

According to the results in Table 9, both examples showed LRV values below 6 within 3 hours of sterilization. However, since the LRV values were found to be in proportion to the sterilization time, it was estimated that the time to reach an LRV of 6 was approximately 4 hours for Example 27 and approximately 1.2 hours for Example 28. If the KPS concentration is 0.1 mol/L, it is estimated that the time required to reach an LRV of 6 is at most approximately 3 hours.

These results indicate that even under a mild condition of 40°C, Bacillus atrophaeus spores can be sterilized in approximately 3 hours in a 0.1 mol/L aqueous KPS solution. This is expected to provide not only improved health safety for operators but also significant cost benefits in terms of equipment and operational expenses for sterilization systems.

[Table 9]

| Change in LRV over time at a sterilization temperature of 40°C after heating and cooling | | | | | | |
|---|---|---|---|---|---|---|
| | Bacterial Spore | KPS Concentration (mol/L) | LRV | | | |
| Example 27 | B. atro. | 0 . 0 5 | 0 h | 1 h | 2 h | 3 h |
| | | | 0 | 1.2 | 4.2 | 5.4 |
| Example 28 | B. atro. | 0.2 | 0 min | 20 min | 40 min | 60 min |
| | | | 0 | 1.5 | 4.2 | 4.8 |

(Examples 29 and 30)

As in the previous section, a sterilization test of Bacillus atrophaeus was conducted in aqueous KPS solutions with concentrations of 0.05 mol/L and 0.2 mol/L, respectively, which had been heated and then cooled and maintained at a lower temperature of 25°C. The change in LRV over time was investigated, and the results obtained for Examples 29 and 30 are shown in Table 10. According to the results in Table 10, the LRV in both examples increased linearly with sterilization time. As a result, the time required to reach LRV = 6 was estimated to be approximately 24 hours in Example 29 (KPS concentration: 0.05 mol/L) and approximately 10 hours in Example 30 (KPS concentration: 0.2 mol/L). If the KPS concentration is 0.1 mol/L, the time required to reach LRV = 6 is estimated to be, at most, about 20 hours.

These results indicate that even at a room temperature of 25°C, sterilization of bacterial spores can be achieved within one day using a KPS aqueous solution at approximately 0.1 mol/L, which is expected to offer great benefits not only for the protection of workers' health, but also for reducing equipment and operational costs of sterilization systems.

[Table 10]

| Change in LRV over time at a sterilization temperature of 25°C after heating and cooling | | | | | | | |
|---|---|---|---|---|---|---|---|
| | Bacterial Spore | KPS Concentration (mol/L) | LRV | | | | |
| Example 29 | B. atro. | 0.05 | 0 h | 1 h | 2 h | 3 h | 6 h |
| | | | 0 | - | 0.3 | 0.7 | 1.0 |
| Example 30 | B. atro. | 0.2 | 0 h | 1 h | 2 h | 3 h | 5 days |
| | | | 0 | 0.4 | 1.2 | 1.8 | 6.6 |

(Corrosion Resistance of Various Materials in KPS Aqueous Solution at 90°C)

(Example 31 to Example 44)

As described above, the decomposing agent aqueous solution of the present invention has excellent performance not only in disinfecting infectious and non-infectious microorganisms but also in sterilizing bacterial spores. However, it contains an oxidizing agent as one of its components. Therefore, in carrying out disinfection and sterilization treatment, it is desirable that the materials of the objects to be treated (such as instruments, containers, and devices) to which infectious and non-infectious microorganisms or bacterial spores adhere or adsorb, have excellent resistance to deterioration (corrosion resistance) against the decomposing agent aqueous solution over a wide temperature range from room temperature to high temperature (90°C). Accordingly, corrosion resistance tests were conducted at 90°C, where oxidative degradation is most concerning, on various materials as Examples 31 to 44. The conditions and results are shown in Table 11.

(Example 31)

A sample (3.977 g) cut from SUS304 stainless steel plate was placed into a KPS aqueous solution (0.1 mol/L) at 90°C in a heat-resistant glass vial, and a corrosion test was conducted for 60 minutes. After the test, the sample was ultrasonically cleaned several times in distilled water, then immersed and stirred in ethanol, followed by thorough drying on filter paper. The sample was then weighed. As a result, no change in weight was observed, indicating that the decomposing agent aqueous solution did not corrode the stainless steel plate at all, thereby demonstrating its excellent corrosion resistance. Therefore, it can be concluded that other stainless steels with corrosion resistance equal to or greater than that of SUS304 are also suitable for disinfection and sterilization treatment using the decomposing agent aqueous solution.

(Example 32)

A corrosion resistance test was conducted under the same conditions as in Example 31, except that a test piece (0.259 g) cut from a commercially available TPX beaker (material: PMP, polymethylpentene, capable of autoclave sterilization at 121°C for 20 minutes) was used. As a result, no change in weight or shape of the test piece was observed after treatment. It was thus clarified that the polymethylpentene sample, having excellent corrosion resistance, is suitable for use as a container or the like in disinfection and sterilization treatment using the decomposing agent aqueous solution.

(Example 33)

A corrosion (degradation) test was conducted under the same conditions as in Example 32, except that a test film piece (0.058 g) cut from a commercially available gamma-ray-sterilized beaker (made of translucent polypropylene) was used. As a result, no change in weight or shape of the test piece was observed after treatment. It was thus clarified that the gamma-ray-sterilized polypropylene film sample is a plastic material with excellent corrosion resistance and is suitable as a material for containers and the like used in sterilization treatment with the decomposing agent aqueous solution. Furthermore, for a test piece of crystalline polypropylene (iPP: isotactic polypropylene) with a melting point of approximately 175°C, no change in weight or surface shape was observed in the degradation test using the decomposing agent aqueous solution of the present invention. Based on these results, it can be said that polypropylene is a suitable material for containers and the like used in disinfection and sterilization treatment with the decomposing agent aqueous solution of the present invention.

(Example 34)

A corrosion resistance test was conducted under the same conditions as in Example 32, except that a test film piece (0.022 g) cut from a commercially available Lumirror film (made of PET: polyethylene terephthalate, 100 $\mu$m thick, T60 transparent grade) was used.
As a result, there was no change in the weight or shape of the test film after treatment, and it was confirmed that the PET film is a suitable material that does not undergo degradation by the decomposing agent aqueous solution of the present invention.

(Example 35)

A corrosion resistance test was conducted under the same conditions as in Example 32, except that a test sheet piece (0.406 g) cut from a commercially available Nafron sheet (PTFE: polytetrafluoroethylene sheet, 1.0 mm thick) was used.
As a result, there was no change in the weight or shape of the test sheet after treatment, and it was confirmed that the PTFE sheet is a suitable material that does not undergo degradation by the decomposing agent aqueous solution of the present invention.

(Example 36)

A corrosion resistance test was conducted using commercially available bleached cotton (dry weight: 0.154 g) in a KPS aqueous solution (concentration: 0.1 mol/L) at 90°C for a reaction time of 60 minutes. After the reaction, the sample was washed five times with distilled water at room temperature, filtered, and its dry weight was measured to be 0.147 g. Due to a slight loss of fibrous powder during the washing and filtration process, the weight decreased by approximately 4.5%. However, the recovered sample was pure white and showed no oxidative degradation. From these results, it was confirmed that bleached cotton is stable in KPS aqueous solution at 90°C.

(Example 37)

A corrosion resistance test was conducted under the same conditions as in Example 36, except that commercially available cosmetic cotton (dry weight: 0.205 g) was used. As a result, no oxidative degradation occurred, similarly to Example 36. However, due to the slight loss of cleansing powder during the washing and filtration process, a weight decrease of 2.0% was observed. From these results, it was confirmed that, like bleached cotton, cosmetic cotton is also stable in KPS aqueous solution at 90°C for 60 minutes.

(Example 38)

A corrosion resistance test was conducted under the same conditions as in Example 36, except that futon cotton made of 100% natural silk fiber (dry weight: 0.138 g) was used. As a result, as in Example 36, the recovered sample was pure white and no oxidative degradation occurred at all. However, a weight loss of 3.2% occurred due to the slight loss of silk fiber powder during the washing and filtration process. This result indicates that the futon cotton was also stable in the 0.1 mol/L KPS aqueous solution at 90°C for 60 minutes.

(Example 39)

A corrosion resistance test was conducted under the same conditions as in Example 31, except that a test sheet (0.654 g) cut from a commercially available natural rubber sheet (1.0 mm thick, black) was used. As a result, no change in the weight of the test sheet was observed after the treatment. It was confirmed that the natural rubber sheet is a suitable material that does not degrade in the decomposing agent aqueous solution of the present invention.

(Example 40)

A corrosion resistance test was conducted under the same conditions as in Example 31, except that a test sheet (0.544 g) cut from a commercially available nitrile rubber sheet (1.0 mm thick, black) was used. As a result, no change in the weight of the test sheet was observed after the treatment. It was confirmed that the nitrile rubber sheet is a material that does not degrade in the decomposing agent aqueous solution of the present invention.

(Example 41)

A corrosion resistance test was conducted under the same conditions as in Example 31, except that a test sheet cut from a commercially available silicone rubber sheet (1.0 mm thick, transparent) weighing 0.473 g was used. As a result, there was no change in the weight of the test sheet due to the treatment, and it was confirmed that the silicone rubber sheet is a material that does not deteriorate in the decomposing agent aqueous solution of the present invention.

(Example 42)

A corrosion resistance test was conducted under the same conditions as in Example 31, except that an aramid (Kevlar®) fiber (Kevlar DP-1, an aromatic polyamide, dry weight: 0.026 g) was used. As a result, there was no change in the weight of the test fiber due to the treatment, and it was confirmed that the aramid (Kevlar®) fiber is a material that does not deteriorate in the decomposing agent aqueous solution of the present invention. Therefore, it can be said that this solution is also applicable to products made of aramid (Kevlar®) fiber composites.

(Example 43)

A corrosion resistance test was conducted under the same conditions as in Example 38, except that a carbon fiber (Tenax HTS40, dry weight: 0.070 g) was used. As a result, there was no change in the weight of the test fiber due to the treatment, and it was confirmed that the carbon fiber is a material that does not deteriorate in the decomposing agent aqueous solution of the present invention. Therefore, it can be said that this solution is also applicable to products made of carbon fiber composites.

(Example 44)

A corrosion resistance test was conducted by dispersing 0.198 g of commercially available pulp fiber in a 0.1 mol/L KPS aqueous solution under harsh conditions at 90°C for 60 minutes. After washing and filtering, the pulp fiber was recovered. As a result, there was no change in the weight of the recovered pulp fiber, and SEM (scanning electron microscope) images of the fiber surface before and after the sterilization treatment showed no difference. Therefore, it was confirmed that pulp fiber, like the solid materials described in Examples 31 to 43, is suitable for disinfection and sterilization of a wide range of products and materials on which infectious or non-infectious microorganisms and bacterial spores may be attached or adsorbed.

[Table 11]

| | | Material | Form | $K_2S_2O_8$ (mol/L) | Reaction Temperature (°C) | Reaction Time (min) | Sample weight (g) | Weight change (after reaction) |
|---|---|---|---|---|---|---|---|---|
| | Example 31 | SUS304 | Steel plate | 0.1 | 90 | 60 | 3.990 | No change |
| | Example 32 | PMP | Sheet | 0.1 | 90 | 60 | 0.259 | No change |
| | Example 33 | PP | Film | 0.1 | 90 | 60 | 0.058 | No change |
| | Example 34 | PET | Film | 0.1 | 90 | 60 | 0.022 | No change |
| | Example 35 | PTFE | Sheet | 0.1 | 90 | 60 | 0.406 | No change |
| | Example 36 | Bleached cotton | Fiber | 0.1 | 90 | 60 | 0.154 | 4.5 wt% decrease |
| | Example 37 | Cosmetic cotton | Fiber | 0.1 | 90 | 60 | 0.204 | 2.0 wt% decrease |
| | Example 38 | Silk | Quilted cotton | 0.1 | 90 | 60 | 0.138 | 3.2 wt% decrease |
| | Example 39 | Natural rubber | Sheet | 0.1 | 90 | 60 | 0.654 | No change |
| | Example 40 | Nitrile rubber | Sheet | 0.1 | 90 | 60 | 0.544 | No change |
| | Example 41 | Silicone rubber | Sheet | 0.1 | 90 | 60 | 0.473 | No change |
| | Example 42 | Kevlar fiber | Fiber | 0.1 | 90 | 60 | 0.026 | No change |
| | Example 43 | Carbon fiber | Fiber | 0.1 | 90 | 60 | 0.070 | No change |
| | Example 44 | Pulp | Fiber | 0.1 | 90 | 60 | 0.198 | No change |

Corrosion Resistance Test of Various Materials against KPS Aqueous Solution
Note 1: Abbreviations of test materials - SUS304: Stainless steel, PMP: Polymethylpentene, PP: Polypropylene, PET: Polyethylene terephthalate, PTFE: Polytetrafluoroethylene
Note 2: Decomposing agent aqueous solution: Potassium peroxodisulfate 0.1 mol/L
Note 3: The weight losses observed in Examples 36, 37, and 38 were due to the outflow of minute fibrous powders during the washing process.

Polyurethane resins (PU) used for endoscopic materials include thermoplastic PU elastomers and ether-based PUs with hydrolysis resistance. The present inventors conducted corrosion resistance tests on commercially available thermoplastic PU elastomers and ether-based PUs to evaluate the hydrolysis resistance-a known weakness of PU-in KPS aqueous solution, as Examples 45 to 48.

(Example 45)

First, a fragment of a transparent thermoplastic PU elastomer tube (abbreviated as Transparent PU 1), weighing 0.109 g, was subjected to a corrosion resistance test in a 0.2 mol/L KPS aqueous solution (pH 1) at 70°C for 1 hour. As a result, no change in weight was observed.

(Example 46)

A fragment of Transparent PU 1 weighing 0.201 g was subjected to the same corrosion resistance test as in Example 21, except that the KPS aqueous solution was heated at 90°C for 30 minutes, then cooled to room temperature (25°C), and the reaction time was extended to 120 hours. As a result, there was no change in the pH (remained at pH 1), no weight loss of the test piece, and no change in surface shape or color.

(Example 47)

A corrosion resistance test was conducted in the same manner as in Example 45, except that a fragment of a transparent ether-based PU tube (hereinafter referred to as Transparent PU 2) weighing 0.128 g was used. As a result, no weight loss or surface change of the test piece occurred.

(Example 48)

A corrosion resistance test was conducted in the same manner as in Example 46, except that a fragment of Transparent PU 2 weighing 0.130 g was used. As a result, it was confirmed that, like Transparent PU 1, Transparent PU 2 also maintained pH 1 in the KPS aqueous solution after heating at 90°C for 30 minutes and cooling to room temperature (25°C), making it one of the disinfectant/sterilizing solutions suitable for long-term sterilization tests at room temperature.

Based on the above test results, the present inventors have found that many materials exhibit corrosion resistance, particularly in a colorless, odorless, transparent KPS aqueous solution (pH 1-2) that has been heated at 90°C for 30 minutes and then cooled to 70°C or lower, and furthermore, that such solution has excellent properties for disinfection and sterilization of infectious and non-infectious organisms as well as bacterial spores.

(Disinfection and Sterilization Apparatus)

As described above, the present invention disinfected and sterilized infectious and non-infectious microorganisms at atmospheric pressure and within a temperature range of 0 to 100°C, using an aqueous solution of potassium peroxodisulfate (KPS) or a mixed aqueous solution of potassium peroxodisulfate and an alkaline compound in a container having corrosion resistance to these reagents. After the treatment is completed, neutralization treatment can be performed as necessary, making it possible to obtain a waste liquid that is safe and environmentally friendly. Furthermore, a treatment apparatus can be realized that enables washing, drying, and recovery of the object to be disinfected or sterilized using sterilized purified water within the above-mentioned range of pressure and temperature.

Accordingly, the treatment apparatus includes equipment for preparing and storing the decomposing agent aqueous solution, equipment for performing disinfection and sterilization reactions, equipment for adjusting the temperature of the aqueous solution, and equipment for immersing the object to be sterilized in the decomposing agent aqueous solution (for example, a stainless steel basket), as well as equipment for washing and drying after the treatment. That is, the shape and size of these apparatuses are not particularly limited. Moreover, the materials of containers and the like are preferably those described in the Examples, and are not particularly limited as long as they have corrosion resistance to the peroxodisulfate aqueous solution. One embodiment of the disinfection and sterilization treatment system of the present invention is described below as Example 49.

(Example 49)

One example of the disinfection and sterilization apparatus of the present invention is illustrated in the schematic diagram below. That is, in accordance with the embodiment of the present application, the object to be disinfected and sterilized (2) is immersed in a predetermined peroxodisulfate aqueous solution (1), and disinfection and sterilization are carried out by a predetermined method to obtain the treated solution (3). Thereafter, the treated solution (3) is subjected to washing, filtration, and drying by a predetermined method to obtain the recovered product (4).

**Claims**

**1.** A decomposing agent aqueous solution for disinfecting and sterilizing microorganisms,

the solution comprising a peroxodisulfate compound as an oxidizing agent,
wherein the solution has a pH in the range of 1 to 2 at a temperature of 20°C to 70°C.

2. The decomposing agent aqueous solution according to claim 1,
   further comprising an alkaline compound.

3. The decomposing agent aqueous solution according to claim 1, wherein the concentration of the peroxodisulfate compound in the aqueous solution is 0.001 mol/L or more and not more than the saturation concentration.

4. The decomposing agent aqueous solution according to claim 1, wherein
   the pH of the aqueous solution three months after its production is maintained at the same pH as at the time of production.

5. A method for producing a decomposing agent aqueous solution,
   comprising heating, under atmospheric pressure, an aqueous solution containing a peroxodisulfate compound, or an aqueous solution containing the peroxodisulfate compound and an alkaline compound, to a temperature in the range of 90°C to 100°C, and then cooling the solution to a temperature in the range of 20°C to 70°C.

6. The method for producing a decomposing agent aqueous solution according to claim 5,
   including adjusting the pH of the aqueous solution after cooling to be lower than the pH of the solution before heating and to be in the range of 1 to 2.

7. A method for treating infectious and non-infectious microorganisms, comprising a step of performing disinfection and sterilization treatment on a treatment target including infectious and non-infectious microorganisms using the decomposing agent aqueous solution according to any one of claims 1 to 4,
   wherein the infectious and non-infectious microorganisms are either in a state dissolved in water or in a state of being attached or adsorbed to a solid surface that is not corroded by the decomposing agent aqueous solution.

8. A disinfection and sterilization treatment system for treating a treatment target including infectious and non-infectious microorganisms using the decomposing agent aqueous solution according to any one of claims 1 to 4, the system comprising:

   a decomposing agent aqueous solution supply unit that supplies the decomposing agent aqueous solution prepared under predetermined conditions;
   a disinfection and sterilization treatment unit that immerses the treatment target in the decomposing agent aqueous solution and performs disinfection and sterilization treatment by a predetermined method;
   a solution recovery unit that recovers the solution after the disinfection and sterilization treatment; and
   a treatment target recovery unit configured to wash and dry the treatment target and recover the treatment target,
   wherein the decomposing agent aqueous solution prepared in the decomposing agent aqueous solution supply unit is recovered by the solution recovery unit after the treatment, and
   the treatment target, after being treated in the disinfection and sterilization treatment unit, is recovered by the treatment target recovery unit.

# FIG.1

## INTERNATIONAL SEARCH REPORT

<table>
<tr><td>International application No.</td></tr>
<tr><td>PCT/JP2024/001894</td></tr>
</table>

**A. CLASSIFICATION OF SUBJECT MATTER**

*A01N 59/02*(2006.01)i; *A01P 3/00*(2006.01)i; *A61L 2/18*(2006.01)i; *C01B 15/08*(2006.01)i; *C11D 3/395*(2006.01)i
FI: A01N59/02 Z; A61L2/18; A01P3/00; C01B15/08; C11D3/395

According to International Patent Classification (IPC) or to both national classification and IPC

**B. FIELDS SEARCHED**

Minimum documentation searched (classification system followed by classification symbols)

A01N59/02; A01P3/00; A61L2/18; C01B15/08; C11D3/395

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 10-95602 A (NIPPON PEROXIDE KK) 14 April 1998 (1998-04-14) paragraphs [0001], [0003], [0013], [0015], examples | 1-7 |
| Y | | 1-4, 7 |
| A | | 8 |
| X | JP 2001-521982 A (THE PROCTER & GAMBLE COMPANY) 13 November 2001 (2001-11-13) paragraphs [0002]-[0005], [0062]-[0064], [0124], examples | 1-4, 7 |
| Y | | 1-4, 7, 8 |
| Y | JP 2001-120876 A (LION CORPORATION) 08 May 2001 (2001-05-08) claims, paragraph [0005], fig. 1-3 | 8 |
| A | | 1-7 |

☑ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 March 2024 | 19 March 2024 |

| Name and mailing address of the ISA/JP | Authorized officer |
|---|---|
| Japan Patent Office (ISA/JP) 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**

| International application No. |
| --- |
| **PCT/JP2024/001894** |

**C. DOCUMENTS CONSIDERED TO BE RELEVANT**

| Category* | Citation of document, with indication, where appropriate, of the relevant passages | Relevant to claim No. |
| --- | --- | --- |
| Y | JP 58-117293 A (TANPEI SEIYAKU KK) 12 July 1983 (1983-07-12)<br>    example 3 | 1-4, 7 |
| A | | 5, 6, 8 |
| Y | JP 11-35987 A (KAO CORPORATION) 09 February 1999 (1999-02-09)<br>    claims, examples | 1-4, 7 |
| A | | 5, 6, 8 |

Form PCT/ISA/210 (second sheet) (July 2022)

**INTERNATIONAL SEARCH REPORT**
Information on patent family members

International application No.

**PCT/JP2024/001894**

| Patent document cited in search report | | | Publication date (day/month/year) | Patent family member(s) | Publication date (day/month/year) |
|---|---|---|---|---|---|
| JP | 10-95602 | A | 14 April 1998 | (Family: none) | |
| JP | 2001-521982 | A | 13 November 2001 | WO 1999/023196 A1 p. 1, line 25 to p. 2, line 30, p. 14, line 14 to last line, p. 29, lines 5-10, examples EP 913462 A1 | |
| JP | 2001-120876 | A | 08 May 2001 | (Family: none) | |
| JP | 58-117293 | A | 12 July 1983 | (Family: none) | |
| JP | 11-35987 | A | 09 February 1999 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002519363 T

**Non-patent literature cited in the description**

- Reference Informatio. *17th Revision of the Japanese Pharmacopoeia*, 2414-2416
- Guidelines for Disinfection and Sterilization. HERUSU PUBLISHING CO., INC., 1999
- Method for Confirming Effectiveness in the Treatment of Infectious Waste. Manual for Infectious Waste Treatment Based on the Waste Management Act. Ministry of the Environment, Environmental Regeneration and Material Cycles Bureau, March 2018
- Guidelines for Disinfection and Sterilization. HERUSU PUBLISHING CO., INC., 2020
- *Environmental Science & Technology Letters*, 2017, vol. 4, 154-160
- *Polymer Degradation and Stability*, 2002, vol. 75 (1), 73-83
- *17th Revision of the Japanese Pharmacopoeia, Reference Information*, 2414-2416
- Reference Information. 17th Revision of the Japanese Pharmacopoeia, 2414-2416
- Guideline on Sterilization Assurance in Clinical Practice. the Japanese Society for Medical Instrumentation, 2015, 138-139
- Guidelines for Sterilization Assurance in Medical Settings. Japan Association for the Advancement of Medical Equipment, 2021, 26